# EUROPÄISCHE PATENTANMELDUNG

(11) **EP 1 175 922 A2**
(43) Veröffentlichungstag der Anmeldung: **30.01.2002**
(21) Anmeldenummer: 01118308.4
(22) Anmeldetag: 27.07.2001
(51) Int. Cl.: A63B 21/055

(54) **Elastisches Übungsgerät**

(30) Priorität: 27.07.2000 DE 10037113
(71) Anmelder: POTAK, Sandor, 63743 Aschaffenburg (DE)
(72) Erfinder: Potak, Sandor, 63743 Aschaffenburg (DE); Petra, Elke, 85646 Anzing (DE)
(74) Vertreter: Petra, Elke, Dipl.-Ing.

(57) **Zusammenfassung**

Es ist ein elastisches Übungsgerät, insbesondere elastisches Übungsseil beschrieben, mit verschiedenenfarbener, geometrischer Oberflächenmusterung. Erfindungswesentlich ist, daß das in Dehnrichtung bzw. axialer Erstreckung auf dem Seil aufgebrachte Muster, z.B. Rhomben (2) oder Quer-Ellipsen (10), in optimal bzw. maximal belastetem Zustand des Übungsgerätes zu einem neuen Muster verändert wird, z.B. Quadrat-Muster oder Kreis-Muster (11), was als Indikator für eine maximale bzw. optimale Kraftaufswendung dient.

## Beschreibung

Die Erfindung bezieht sich auf ein elastisches Übungsgerät gemäß Oberbegriff des Anspruchs 1, wie es insbesondere in Form von formelastischen Bändern, Schnüren, Seilen, Ringen, Platten für sportliche bzw. sportmedizinische Zwekke, Verwendung findet.

Als elastische Übungsgeräte sind vorwiegend umwebte, runde, gummielastische Bänder bekannt, mit optisch ansprechender uni oder buntgestalteten Webstrukturen. Diese werden beispielsweise als Expander-Gurt oder als "Spinne" für Gepäckfixierungen an Fahrzeugen usw. eingesetzt. Die farbliche und/oder strukturelle Eigenart der in der Regel geflochtenen Ummantelung dient ausschließlich der Verkaufsförderung und ist oft modebedingt. Entsprechend der Dehnungseigenschaft der jeweiligen Seile und der gewählten optischen Struktur oder des gewählten Musters, beispielsweise Karo-, Streifen-, Hahnentritt- ect. -Muster verändern sich die optischen Erscheingungsformen dieser Strukturen. Diese Strukturveränderungen geben jedoch keinerlei Auskunft über die erzielte Höhe der Belastung bzw. Dehnung der elastischen Bänder, Seile, Matten oder vergleichbarer Produkte. Sollen diese bekannten elastischen Dehnungsprodukte z.B. für sportliche oder sportmedizinische Zwecke, wie Rehabilitationsübungen, mit der Möglichkeit der Belastungsmessung von Muskeln / Muskelgruppen verwendet werden, sind entsprechende Meßeinrichtungen, wie Meßdosen oder Zugfederwaagen zu verwenden. Hierdurch werden jedoch die ansonsten einfachen und praktischen Übungsgeräte verkompliziert, wodurch deren Handhabung schwieriger und die Herstellkosten höher werden.

So zeigt die US 5,816,984 A ein elastisches Übungsgerät, dessen elastische Elemente auch textilumwoben sein können und auf dessen Oberfläche Muster aufgetragen oder eingewebt sind, die sich bei belastetem Zustand ändern. So werden aus einem dicken Strich bzw. einem dicken Ring bei maximaler Belastung mehrere parallele Striche bzw. aus einem Umfangsring mehrere parallele Ringe oder aus mehreren Strichen mehrere Buchstaben, die ein Wort ergeben. Die sich ergebenden unterschiedlichen Figuren zeigen jedoch nur eine mehr oder weniger starke Belastung an, können nicht relativ genau eine vorbestimmte Belastung erkennen lassen, bei der der Übende z.B. aufhören soll, die Zugkraft zu erhöhen. Ganz wichtig ist aber, die Zugkraft über einen längeren Zeitabschnitt konstant zu halten, z.B. 60 Sekunden, um ein optimales Trainingsresultat zu erhalten (isotonisch/isomerisch). Der Übende sieht zwar an seinem Übungsgerät, daß er fleißig belastet, nicht jedoch, wann genau er mit der Belastungsvergrößerung aufhören soll.

Aufgabe der Erfindung ist es, ein elastisches Übungsgerät o. g. Gattung anzugeben, mit dem es möglich ist, auch ohne den Einsatz entsprechender Meßgeräte, in einfacher Weise dessen optimalen Dehnungszustand optisch relativ genau erkennen zu lassen.

Diese Aufgabe wird durch ein elastisches Übungsgerät mit den kennzeichnenden Merkmalen des Anspruchs 1 gelöst. Vorteilhafte Ausgestaltungen sind den Unteransprüchen zu entnehmen.

Demgemäß ist das elastische Übungsgerät, z.B. Bänder, Schnüre, Seile, Ringe, Platten in Dehnrichtung, im wesentlichen auf der gesamten Dehn-Länge mit einem Längs-Muster, vorzugsweise einem geometrischen Zick-Zack- oder Wellenmuster versehen, das in unbelastetem/ungedehntem Zustand einen bestimmten Winkel- oder Linienstand aufweist, während bei Dehnung mit vorbestimmter Kraft, d.h. in maximal/optimal belastetem Zustand, der z.B. einer Belastung von ca. 75% entspricht, ein anderes, vorbestimmtes, als solches gut erkennbares Muster gebildet wird bzw. sich ergibt, das als nicht zu über- oder unterschreitender Belastungsindikator dient.

Somit können Bänder, Schnüre, Seile, Matten, Ringe oder beliebig gestaltete andere elastische Produkte, optimal für sportliche oder sportmedizinische Zwecke, wie Rehabilitationsübungen, mit der Möglichkeit der zumindest groben Belastungserkennung von Muskeln bzw. Muskelgruppen verwendet werden. Die elastischen Übungsgeräte sind optisch so gestaltet, daß sie gleichzeitig ihre eigenen Kraftmeßgeräte darstellen. Diese Geräte können z.B. auch zum Fixieren oder Heben von Gegenständen, Gewichten etc. verwendet werden, wobei dann die Musterung z.B. die maximale Belastung beim Heben erkennen läßt, also gleichzeitig als Sicherheitsindikator z. B. beim Lastenheben dient. Dieser besondere Sicherheitsaspekt ist bei Sportgeräten, insbes. bei schulischen Sportgeräten von äußerster Wichtigkeit . Das Muster-Warnsignal hat dort Unfallvorsorge-Funktion und verhindert z. B. ein Ausreißen der Gerät-Seile aus den Verankerungen, wenn durch mehrere Kinder mit entsprechend großer Kraft daran gezogen wird.

Bei umwebten elastischen Geräten wird vorzugsweise die Möglichkeit genutzt, verschiedenfarbige Mikrofäden der Ummantelung zu einer - meistens vorab berechneten -Deformationsstruktur zu verwenden. So können z.B. in axialer Aneinanderreihung Längs- oder Querrhomben, Doppel-Sinuslinien, Ellipsen, Loch-Ringe oder Umfangslinien, die in bestimmtem Umfangs-Abstand zueinander aufhören, verwendet werden. Bei vorbestimmter Kraftaufwendung bzw. Dehnung verändern sich diese Ornamente dann soweit, daß z.B. aus den Rhomben oder Sinuslinien axial verlaufende parallele Linien, oder Quadrate, aus den umfangsmäßig unterbrochenen Kreislinien durchgehende Kreise, aus den querovalen Ellipsen, runde Kreise und aus den Loch-Ringen breite Ringe werden. Jeweils bei Erreichen des entsprechenden angestrebten, neuen Bildes bzw. Figur, wie Doppellinie, Umfangskreis, breite Ringe oder axial aneinanderliegend angeordnete Kreise oder Quadrate, weiß derjenige, der die Dehnkraft auf das Gerät aufbringt, daß die maximale oder optimale Dehnung erreicht ist und eine weitere Erhöhung der Zugkraft zu unterlassen ist.

In gleicher Weise verhält es sich bei nicht umwebten elastischen Produkten, wie elastischen Flachbändem, Schnüren, Seilen, Platten und Ringen. Bei diesen kann die optische Struktur bzw. Musterung mit Hilfe bekannter Farbauftragverfahren mit geeigneten elastischen Druckfarben erfolgen. Es ist jedoch auch ein Einbrennen z.B. mit Lasertechnologie, möglich.

Nachfolgend wird die Erfindung anhand mehrerer Ausführungsbeispiele unter bezug auf die Zeichnung näher erläutert.

Es zeigen:
- Fig. 1:: eine teilweise Draufsicht auf ein Seil mit Rauten-Längs-Rhomben-Muster, in ungedehntem Zustand,
- Fig. 2:: eine Draufsicht wie in Fig. 1, mit Doppelsinus-Muster, in ungedehntem Zustand,
- Fig. 3:: eine Draufsicht auf die Geräte nach Fig. 1 und 2, in gedehntem Zustand, ein Doppellinien-Muster zeigend,
- Fig. 4:: eine Teil-Draufsicht eines Seil-Gerätes, mit Quadraten und Umfangs-Teilkreisbögen, in ungedehntem Zustand,
- Fig. 5:: eine Draufsicht auf das Gerät nach Fig. 4, in gedehntem Zustand,
- Fig. 6:: eine Draufsicht auf ein Gerät mit Ellipsen-Muster, in ungedehntem Zustand,
- Fig. 7:: eine Draufsicht auf das Gerät aus Fig. 6, in gedehntem Zustand mit Kreis-Muster,
- Fig. 8:: eine Draufsicht auf ein Gerät mit Quer-Rhomben-Muster in ungedehntem Zustand,
- Fig. 9:: eine Ansicht wie in Fig. 8, in gedehntem Zustand, mit Quadrat-Muster,
- Fig. 10:: eine Draufsicht auf ein Gerät mit Loch-Ring-Muster, in ungedehntem Zustand, und
- Fig. 11:: eine Ansicht wie in Fig. 10, in gedehntem Zustand mit Ring-Muster.

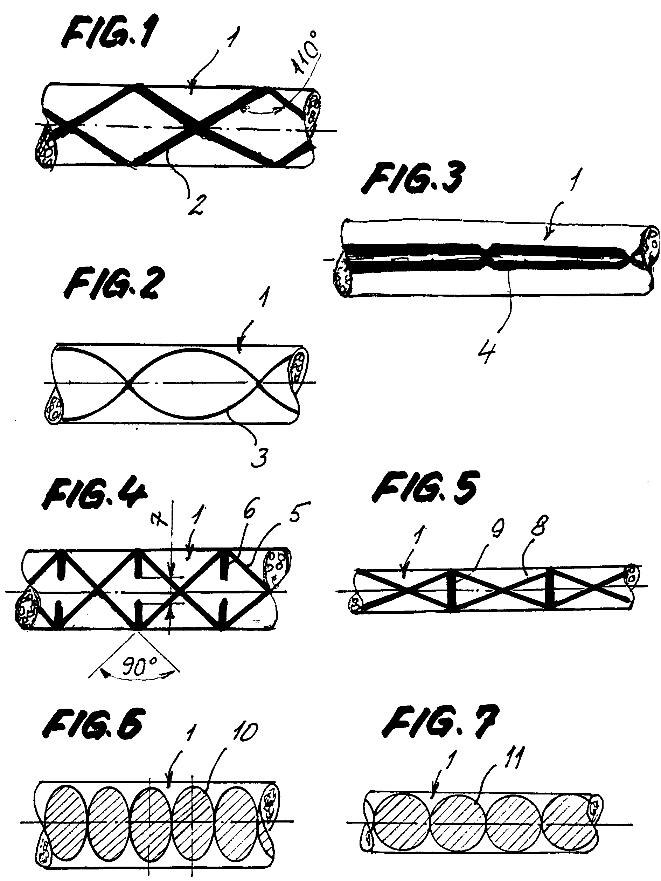

Bei dem in Fig. 1 dargestellten Ausführungsbeispiel ist ein Gerät 1, hier z.B. ein elastisches Seil, mit einem Längs-Rhomben-Muster 2 versehen. Die mit ihrer größeren Achse in Längs- bzw. Achsausrichtung angeordneten Rhomben des Musters besitzen einen in Umfangsrichtung weisenden stumpfen Winkel von z.B. 110°.

Bei dem in Fig. 2 dargestellten Ausführungsbeispiel ist das Seil 1 mit einem Doppel-Sinus-Muster 3 versehen.

Fig. 3 zeigt das Seil 1 in so weit gespanntem Zustand, daß z.B. die Rhomben oder Doppel-Sinus-Muster 2 bzw. 3 aus Fig. 1 bzw. 2 zu axial ausgerichteten Doppellinien 4 deformiert bzw. gezogen wurden.

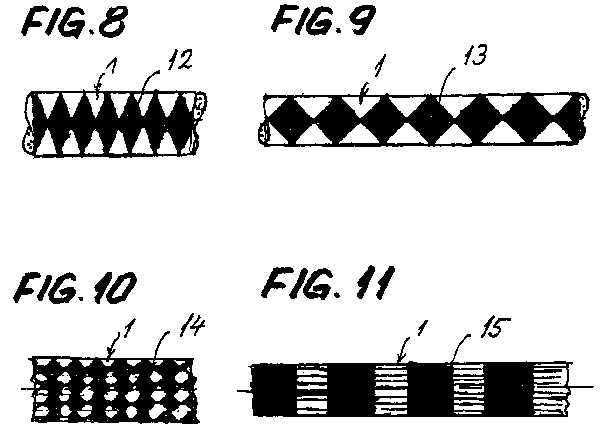

Fig. 4 zeigt ein Quadrat-Muster 5, bei dem diagonal, d.h. in Umfangsrichtung verlaufend, Bogen-Umfangslinien 6 vorgesehen sind, die zur Mittelachse des Quadrat-Musters zueinander um einen Betrag 7 beabstandet sind.

Aus Fig. 5 ist zu erkennen, daß in belastetem Zustand aus dem Quadrat-Muster 5 ein Längs-Rhomben-Muster 8 geworden ist, während die unterbrochenen Umfangslinien 6 sich soweit umfangsmäßig genähert haben, daß sie zu einer durchgehenden Linie bzw. einem Umfangskreis 9 verschmolzen sind.

In Fig. 6 zeigt das seilförmige Gerät 1 ein Ellipsen-Muster mit in axialer Aneinanderreihung befindlichen Ellipsen, wobei die langen Achsen der Ellipsen jeweils in Umfangsrichtung zeigen.

Fig. 7 zeigt wie bei entsprechender Belastung des in Fig. 6 dargestellten Seiles, aus den Ellipsen 10 Kreise 11 geformt werden.

Beim Ausführungsbeispiel nach Fig. 8 ist ein Quer-Rhomben-Muster (12) vorgesehen, das, wie aus Fig. 9 ersichtlich, bei entsprechender Dehnung ein Quadrat-Muster 15 ergibt.

Schließlich zeigt Fig. 10 ein Loch-Muster 14, mit am Umfang ringförmig angeordneten Löchern bzw. punktförmigen Farbgebungen, wobei sich bei entsprechender Zug-Belastung ein Ring-Muster 15 ergibt, mit axial aufeinander folgenden, relativ breiten Ringen abwechselnder Farbe, die optisch als Quadrate wirken, wie aus Fig. 11 ersichtlich ist.

### Bezugszeichenliste

- 1.: Gerät / Seil
- 2.: Längs-Rhomben-Muster
- 3.: Doppel-Sinus-Muster
- 4.: Doppellinien
- 5.: Quadrat-Muster
- 6.: Umfangs-Bogenlinien
- 7.: Abstand
- 8.: Längs-Rhomben
- 9.: Umfangskreis
- 10.: Ellipsen
- 11.: Kreis-Muster
- 12.: Quer-Rhomben
- 13.: Quadrat
- 14.: Loch-Muster
- 15.: Ring-Muster

## Patentansprüche

1. Elastisches Übungsgerät, insbesondere Band, Schur, Seil, Ring oder Platte, mit an der Oberfläche vorhandenem Muster, das in unbelastetem Zustand ein anderes Muster darstellt als in belastetem Zustand,
**dadurch gekennzeichnet,**
**daß** das in Dehnrichtung, im wesentlichen auf dessen gesamten Länge aufgebrachte Muster ein geometrisches Zick-Zack-, Wellen- oder Flächenmuster ist, das in unbelastetem/ungedehntem Zustand einen bestimmten Winkel- oder Linienstand aufweist, während bei Dehnung mit vorbestimmter Kraft, d.h. bei maximal/optimal belastetem Zustand, der z.B. einer Belastung von ca. 75% entspricht, sich ein anderes, vorbestimmtes, als solches gut erkennbares Muster bildet bzw. ergibt, das als nicht zu über- oder unterschreitender Belastungsindikator dient.

2. Übungsgerät nach Anspruch 1, **dadurch gekennzeichnet,**
**daß** ein Längs-Rhomben-Muster (2) vorgesehen ist, das aus entlang ihrer Längsachse in Dehnrichtung aneinandergereihten Rhomben besteht, deren stumpfen Winkel jeweils ca. 110° betragen, wobei in optimaler Dehnungs-Situation von z.B. 75% die Muster-Linien im wesentlichen zu einer axial ausgerichteten Doppellinie (4) verschoben werden.

3. Übungsgerät nach Anspruch 1, **dadurch gekennzeichnet,**
**daß** ein Quer-Rhomben-Muster (12) vorgesehen ist, das aus entlang ihrer kurzen Achse in Dehnrichtung aneinandergereihten Rhomben besteht, die in vorbestimmter Dehnsituation zu Quadraten (13) werden.

4. Übungsgerät nach Anspruch 1, **dadurch gekennzeichnet,**
**daß** ein Quadrat-Muster (5) vorgesehen ist, mit diagonaler Aneinanderreihung der Quadrate, wobei aus den jeweils in Umfangsrichtung weisenden freien Quadratecken diagonal einwärts, aufeinander zuweisende Umfangs-Linien (6) vorgesehen sind, die in ungedehntem Zustand in Abstand (7) zur Mitte bzw. zueinander aufhören und daß unter Zugbelastung die Quadrate (5) zu Rhomben (8) auseinandergezogen sind und die Innenenden der Umfangs-Diagonallinien optisch aufeinandertreffen.

5. Übungsgerät nach Anspruch 1, **dadurch gekennzeichnet,**
**daß** ein Doppel-Sinus-Muster (3) vorgesehen ist, das in gedehntem Zustand zu einer Doppellinie (4) wird.

6. Übungsgerät nach Anspruch 1, **dadurch gekennzeichnet,**
**daß** ein Ellipsen-Muster (10) vorgesehen ist, bestehend aus in Dehnrichtung über ihre kurze Achse aneinandergereihten Ellipsen, deren Längsachsen jeweils in Umfangs- bzw. Tangentialrichtung weisen und daß in maximalem bzw. optimalem Dehnzustand die Ellipsen (10) zu Kreisen (11) verformt sind.

7. Übungsgerät nach Anspruch 1, **dadurch gekennzeichnet,**
**daß** ein Loch-Muster (14) vorgesehen ist, mit axial zu einander beabstandeten Umfangs-Loch-Ringen und daß unter max. bzw. bestimmter Zugbelastung ein Ring-Muster (15) entsteht, mit in Dehnrichtung aufeinanderfolgenden, breiteren Ringen, die optisch als Quadrate wirken.

8. Übungsgerät nach Anspruch 1, **dadurch gekennzeichnet,**
**daß** es in bezug auf die maximale Zugkraft und das unter deren Einwirkung entstehende, neue Muster, wie aus Querrhomben entstandene Diagonal-Quadrate, genau berechnet ausgelegt ist, einen Sicherheitsindikator bzw. ein Muster-Warnsignal beim Heben von Lasten bzw. an Sportgeräten darstellend.

9. Übungsgerät nach den Ansprüchen 1 bis 3, **dadurch gekennzeichnet, daß** das Gerät ein umwebtes elastisches Seil ist, bei dem verschiedene, farbige Microfäden der Ummantelung zu einer vorab berechneten Deformationsstruktur verwendet werden.

10. Übungsgerät nach den Ansprüchen 1 bis 4, **dadurch gekennzeichnet, daß** bei nicht umwebten bzw. umflochtenen elastischen Geräten, wie elastischen Flachbänder, Schüren, Seilen, Platten, Ringe, die optischen Strukturen mit z.B. durch Bedrucken aufgebrachter Farben, oder durch Einbrennen z.B. über Lasertechnik, erreicht bzw. aufgebracht sind.
